# FASCICULE DE BREVET EUROPEEN

(11) **EP 0 939 654 B1**
(45) Date de publication et mention de la délivrance du brevet: **21.04.2004**
(21) Numéro de dépôt: 97940183.3
(22) Date de dépôt: 05.09.1997
(51) Int. Cl.: A61K 45/06, A61P 9/10

(54) **UTILISATION DES INHIBITEURS DE NO SYNTHASE ET DES PIEGEURS DES FORMES REACTIVES DE L'OXYGENE POUR LE TRAITEMENT DE L'ISCHEMIE**
VERWENDUNG VON NO SYNTHASE HEMMERN UND FÄNGERN VON REAKTIVEN SAUERSTOFFSFORMEN ZUR BEHANDLUNG VON ISCHÄMIE
USE OF NO SYNTASE INHIBITORS WITH SCAVENGERS OF OXYGEN REACTIVE FORMS FOR THE TREATMENT OF ISCHEMIA

(30) Priorité: 06.09.1996 FR 9610875
(43) Date de publication de la demande: 08.09.1999
(73) Titulaire: SOCIETE DE CONSEILS DE RECHERCHES ET D'APPLICATIONS SCIENTIFIQUES (S.C.R.A.S.), 75016 Paris (FR)
(72) Inventeur: CHABRIER de LASSAUNIERE, Pierre-Etienne, F-75016 Paris (FR); BIGG, Denis, F-91190 Gif sur Yvette (FR)
(74) Mandataire: Bourgouin, André
(86) Numéro de dépôt international: PCT/FR1997/001567
(87) Numéro de publication internationale: WO 1998/009653

(56) Documents cités:
- GALLEY H F ET AL: "Regulation of nitric oxide synthase activity in cultured human endothelial cells: Effect of antioxidants" FREE RADICAL BIOLOGY & MEDICINE, 21 (1). 06-1996. 97-101., XP000672950
- OHOI I.; TAKEO S.: 'Involvement of SOD and NO in the genesis of reprefusion arrhythmias in rats' EUROPEAN J. OF PHARMACOL. vol. 306, pages 123 - 131
- KEUSCH G.T.: 'Antioxidants in infection' J. NUTR. SCI. VITAMINOL. vol. 39, 1993, pages S23 - S33, XP000996268

## Description

L'invention concerne une composition pharmaceutique comprenant, à titre de principe actif, au moins une substance inhibitrice de NO synthase et au moins une substance piègeur de formes réactives de l'oxygène, et éventuellement un support pharmaceutiquement acceptable. L'invention concerne également un produit comprenant au moins une substance inhibitrice de NO synthase et au moins une substance piègeur de formes réactives de l'oxygène en tant que produit de combinaison, sous forme séparée, de ces principes actifs.

Une composition pharmaceutique et un produit selon l'invention sont intéressants dans le traitement de pathologies où le monoxyde d'azote et les formes réactives de l'oxygène sont impliquées, et notamment :

Dans l'ensemble de ces pathologies, il existe des évidences expérimentales démontrant l'implication du monoxyde d'azote ou des formes réactives de l'oxygène (Kerwin et al., Nitric oxide : a new paradigm for second messengers, J. Med. Chem. 38, 4343-4362, 1995 ; Halliwell B., Gutteridge JMC., Free radicals in biology and medicine, 2nd ed., Oxford, Clarendon Press, 1989). C'est le cas notamment de l'infarctus cérébral expérimental qui illustre l'invention (Buisson et al, The neuroprotective effect of a nitric oxide inhibitor in a rat model of focal ischemia., Br J Pharmacol. 106, 766-767, 1992 ; Nowicki et al, Nitric oxide mediates neuronal cell death after focal cerebral ischemia in the mouse, Eur. J. Pharmacol. 204, 339-340, 1991 ; Zhao et al, Delayed treatment with the spin trap α-phenyl-N-tert-butyl nitrone (PBN) reduces infarct size following transient middle cerebral artery occlusion in rats, Acta. Physiol. Scand., 1994 ; Schulz et al, Improved therapeutic window for treatment of histotoxic hypoxia with a free radical spin trap, J. Cereb. Blood Flow Metabol. 15, 948-952 (1995)). Dans ce contexte, les médicaments pouvant inhiber la formation du monoxyde d'azote ou des formes réactives de l'oxygène peuvent apporter des effets bénéfiques. Aucune association de ces deux principes actifs, à savoir un inhibiteur de NO synthase et un piègeur de formes réactives de l'oxygène, n'a été réalisée. Comme cela est exposé dans la partie expérimentale, ces deux principes actifs agissent de manière synergique. En effet, ces deux principes actifs administrés à des doses subactives (c'est-à-dire à des doses qui ne produisent pas par elles-mêmes d'effet thérapeutique), produisent, lorsqu'ils sont associés, un effet thérapeutique hautement significatif.

L'avantage de cette association est de diminuer de façon importante les doses de chacun des principes actifs et ainsi de diminuer considérablement leurs effets indésirables tout en gagnant de l'efficacité thérapeutique. Cette invention est particulièrement bien illustrée dans un modèle pathologique expérimental de neurodégénération : l'ischémie cérébrale avec reperfusion.

L'invention a donc pour objet une composition pharmaceutique comprenant, à titre de principe actif, au moins une substance inhibitrice de NO synthase et au moins une substance piègeur de formes réactives de l'oxygène, et éventuellement un support pharmaceutiquement acceptable.

L'invention a plus particulièrement pour objet une composition pharmaceutique comprenant, à titre de principe actif, une substance inhibitrice de NO synthase et une substance piègeur de formes réactives de l'oxygène.

Dans le terme inhibiteur de NO synthase, il faut comprendre tout inhibiteur spécifique ou non spécifique de l'une de ses isoformes qu'elle soit constitutive (neuronale ou endothéliale) ou inductible (Kerwin et al., Nitric oxide : a new paradigm for second messengers, J. Med. Chem. 38, 4343-4362, 1995).

Dans le terme piègeur de formes réactives de l'oxygène, il faut comprendre toute substance chimique ou enzymatique capable de s'opposer ou de pièger les ou l'une des formes réactives de l'oxygène telles que O₂⁻,OH⁻, RO₂⁻, RO⁻, ONO₂⁻, NO⁻, NO₂⁻ ou H₂O₂ (Halliwell B., Gutteridge JMC., Free radicals in biology and medicine, 2nd ed., Oxford, Clarendon Press, 1989). Ces substances peuvent être naturelles ou synthétiques et posséder des propriétés antioxydantes. (Santrucek and Krepelka, Antioxidants - Potential chemotherapeutic agents Drugs Future 13, 975-996, 1988 ; Jackson et al, Antioxidants : a biological defense mechanism for the prevention of atherosclerosis, Med. Res. Reviews 13, 161-182 (1993) ; Aruoma, Characterization of drugs as antioxidant prophylactics, Free Rad. Biol. Med. 20, 675-705 (1996)).

Dans une composition pharmaceutique selon l'invention, l'inhibiteur de NO synthase et le piègeur des formes réactives de l'oxygène peuvent se présenter sous forme séparée ou sous forme combinée en formant un sel. De préférence, le sel est formé à partir d'un dérivé de la substance inhibitrice de NO synthase contenant au moins un groupe basique et d'un dérivé de la substance piègeur de formes réactives de l'oxygène contenant au moins un groupe acide. Ainsi des sels peuvent être formés, selon les méthodes connues de l'homme de l'art, à partir des inhibiteurs de NO synthase comme par exemple les amidines, les guanidines, les pyridines ou les pipéridines telles que définies ci-après, et les piègeurs de formes réactives de l'oxygène comme par exemple les acides phénoliques tels que définis ci-après, et plus particulièrement l'acide 3-5-di-tert-butyl-4-hydroxybenzoique, l'acide caféique, l'acide sinapinique ou l'acide gallique.

L'invention a également pour objet un produit comprenant au moins une substance inhibitrice de NO synthase et au moins une substance piègeur de formes réactives de l'oxygène en tant que produit de combinaison, sous forme séparée, pour une utilisation simultanée ou séquentielle dans le traitement de pathologies dans lesquelles le monoxyde d'azote et les formes réactives de l'oxygène sont impliquées telles que les troubles cardiovasculaires et cérébrovasculaires, les troubles du système nerveux central ou périphérique, les maladies prolifératives et inflammatoires, les transplantations d'organes, les maladies autoimmunes et virales, le cancer et toutes les pathologies caractérisées par une production ou un dysfonctionnement de monoxyde d'azote et/ou des formes réactives de l'oxygène.

Dans une composition pharmaceutique ou un produit selon l'invention, l'inhibiteur de NO synthase et le piègeur de formes réactives de l'oxygène peuvent se présenter à des doses qui peuvent être identiques ou différentes. Les dosages sont choisis en fonction des composés associés à des diluants ou excipients appropriés.

L'inhibiteur de NO synthase et le piègeur de formes réactives de l'oxygène peuvent être administrés de manière simultanée ou séquentielle, par la même voie d'administration ou par des voies différentes, suivant qu'ils se présentent sous forme séparée ou combinée. De préférence, les voies d'administration sont orale, parentérale ou topique.

Parmi les inhibiteurs de NO synthase, on peut définir les composés de type amino-acide et non amino-acide. Les inhibiteurs de NO synthase de type amino-acide peuvent être des composés tels que décrits dans les demandes WO95/00505, WO94/12163, WO96/06076 et EP230037 incorporées par référence dans la présente demande, ou bien des dérivés de la L-arginine, de l'ornithine ou de la lysine tels que décrits dans les demandes WO93/24126, WO95/01972, WO95/24382, WO95/09619 et WO95/22968, incorporées par référence dans la présente demande.

Les inhibiteurs de NO synthase de type non amino-acide, peuvent être des composés de la familles des guanidines, des isothiourées, des nitro- ou cyano-aryles, des amino-pyridines ou amino-pyrimidines, des amidines, des indazoles ou des imidazoles.

Les guanidines inhibiteurs de NO synthase peuvent être les composés tels que définis dans les demandes WO95/28377, WO91/04023, WO94/21621, WO96/18607 et WO96/18608 incorporées par référence dans la présente demande.

Les isothiourées inhibiteurs de NO synthase peuvent être les composés tels que définis dans les demandes WO95/09619, WO96/09286, WO94/12165, WO96/14842, WO96/18607, WO96/18608, WO96/09286, EP717040 et EP718294 incorporées par référence dans la présente demande.

Les nitro- ou cyano-aryles inhibiteurs de NO synthase peuvent être les composés tels que définis dans la demande WO94/12163, incorporée par référence dans la présente demande.

Les amino-pyridines ou amino-pyrimidines inhibiteurs de NO synthase peuvent être les composés tels que définis dans les demandes WO94/14780, WO96/18616 et WO96/18617 incorporées par référence dans la présente demande.

Les amidines inhibiteurs de NO synthase peuvent être les composés tels que définis dans les demandes WO95/11014, WO96/01817, WO95/05363, WO95/11231, WO96/14844 et WO96/19440 incorporées par référence dans la présente demande, ou des composés tels que la N-phényl-2-thiophènecarboximidamide.

Ohoi et al. (European J. Pharmac. (1996) 306, 123-131) décrit l' efft d'une substance piégeur des formes réactives de l'oxygène - la superoxide dismutase, et des substances inhibitrices de la NO synthase - L-NAME et L-NNA (dérives de la L-arginine), sur l' incidence et irréversibilité de la fibrillation ventriculaire chez le rat. Les résultats obtenus dans cette étude démontrent que la superoxide dismutase ainsi que la L-NAME, utilisés individuellement, réduisent l'incidence et l'irréversibilité (mortalité) de la fibrillation ventriculaire. Par contre la L-NNA permet de réduire la mortalité associée a la fibrillation ventriculaire, mais ne réduit pas son incidence. Le traitement simultané avec la superoxide dismutase et L-NAME produit un effet synergetique qui se traduit par une réduction de l'incidence et de l'irréversibilité de la fibrillation ventriculaire supérieur à celle observée avec chacune de ces substances.

Keutsch ( J. Nutr. Sci. Vitaminol. (1993) 39, S23-S33) divulgue le rôle des formes réactives de l'oxygène et des radicaux dérivés du monoxyde d'azote dans la genèse du choc septique. D'après ce document, les inhibiteurs de NO synthase, comme la L-NMA peuvent réverser le choc induit par le LPS et par le TNF; et la superoxide dismutase réduit les effets hypotensifs et la mortalité induites par le LPS et atténue le dommage du poumon dans un modèle expérimental de choc septique induit par E. coli.

Les indazoles inhibiteurs de NO synthase peuvent être des composés de formule générale I_{A} 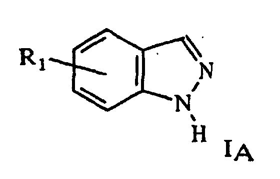 dans laquelle R₁ représente un ou plusieurs substituants choisis parmi l'atome d'hydrogène, le radical nitro, halo, alkyle inférieur ou alkoxy inférieur.

Les imidazoles inhibiteurs de NO synthase peuvent être les composés de formule générale II_{A} 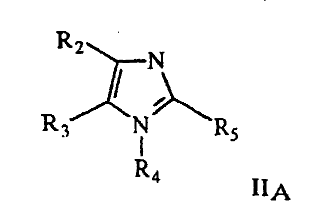 dans laquelle R₂ et R₃ représentent, indépendemment l'atome d'hydrogène, le radical halo, hydroxy, amino, alkyl ou alkoxy, ou R₂ et R₃ sont liés ensembles et forment le radical phényle condensé avec le cycle imidazole, le radical phényle étant éventuellement substitué par un ou plusieurs substituants choisis parmi les radicaux hydroxy, trifluorométhyle, halo, carboxy, alkyle inférieur, alkoxy inférieur ou alkényle inférieur ; R₄ représente un atome d'hydrogène,un radical alkyle inférieur, amino, alkyle inférieur amino ou phényle, le radical phényle étant éventuellement substitué par un ou plusieurs substituants choisis parmi les radicaux hydroxy, trifluorométhyle, halo, carboxy, alkyle inférieur, alkoxy inférieur ou alkényle inférieur ; R₅ représente l'atome d'hydrogène, un radical alkyle inférieur, amino, alkyle inférieur amino.

Les piègeurs de formes réactives de l'oxygène peuvent être choisis parmi l'acide ascorbique, la N-acétyl-cystéine, le β-carotène (Hao Chen et al, Free Radical Biology and Medicine 18 (5), 949-953 (1995)), le coenzyme Q10 (S. Tereao et al., J. Org. Chem., 44, 868 (1979)) ou les composés captodatifs (H.G. Viehe et al., Acc. Res., 18, 148-154 (1985), incorporée par référence dans la présente demande). Les piègeurs de formes réactives de l'oxygène peuvent également être choisis parmi les composés phénoliques, les nitrones, des dérivés de l'indole, des imidazoles ou des carbazoles, ou bien également être les enzymes capables de neutraliser les ou l'une des formes réactives de l'oxygène telles que les superoxydes dismutases, les catalases ou les glutathions peroxydases et leurs mimétiques.

Parmi les composés phénoliques piègeurs de formes réactives de l'oxygène, on peut citer le probucol ; l'ubiquinone ; les dérivés du tocophérol, à savoir l'α-, β-, γ-, ε-, τ- ou δ-tocophérol ou les flavonoïdes phénoliques (R. A. et al, Phytochemistry, 27(4), 969-978 (1988), incorporée par référence dans la présente demande). Les composés phénoliques piègeurs des formes réactives de l'oxygène peuvent également être choisis parmi les composés de formule générale I_{B} ou II_{B} 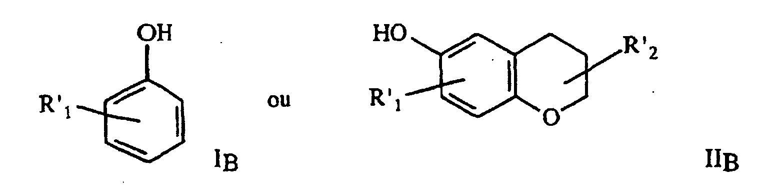 dans laquelle R'₁ représente un ou plusieurs substituants choisis parmi l'atome d'hydrogène, les radicaux hydroxy, halo, carboxy, alkyle inférieure, alkoxy inférieur, alkényle inférieur ou alkoxy carbonyle, les radicaux alkyle, alkoxy et alkényles étant éventuellement substitués par un radical hydroxy, halo, carboxy ou amino ; et R'₂ représente un ou plusieurs substituants choisis parmi l'atome d'hydrogène ou les radicaux alkyle inférieur éventuellement substitué, alkoxy inférieur, hydroxy, halo, amino ou carboxy.

Les nitrones piègeurs de formes réactives de l'oxygène peuvent être les composés tels que définis dans les demandes WO96/15110, WO88/05044 et le brevet US5310916 incorporés par référence dans la présente demande.

Les dérivés de l'indole piégeurs des formes réactives de l'oxygène, peuvent être des composés de formule générale III_{B} 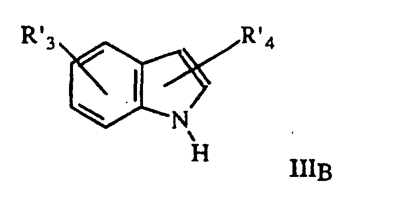 dans laquelle R'₃ représente un ou plusieurs substituants choisis parmi l'atome d'hydrogène, les radicaux hydroxy, halo, alkyle inférieur ou alkoxy inférieur ; R'₄ représente un ou plusieurs substituants choisis parmi l'atome d'hydrogène, les radicaux halo, hydroxy, amino, carboxy ou alkylcarbonylaminoalkyle.

Les dérivés de l'indole piégeurs des formes réactives de l'oxygène, peuvent être également des composés tels que définis dans la demande WO96/26941.

Parmi les imidazoles piégeurs de formes réactives de l'oxygène, on peut citer de préférence l'imidazole elle-même ou la cimétidine.

Parmi les carbazoles piégeurs de formes réactives de l'oxygène, on peut citer le 4-hydroxycarbazole ou le carvedilol.

Tel qu'il est utilisé ici, le terme inférieur en référence aux groupes alkyle et alkoxy désigne des groupes hydrocarbonés aliphatiques saturés, linéaires, ou ramifiés, comportant de 1 à 6 carbones comme, par exemple, méthyle, éthyle, propyle, isopropyle, butyle, t-butyle, méthoxy et éthoxy. En référence aux groupes alkényle, le terme inférieur désigne des groupes comportant 2 à 6 atomes de carbone et une ou plusieurs doubles ou triples liaisons comme, par exemple, les groupes vinyle, allyle, propènyle, isopropènyle, pentènyle, butènyle, hexanyle, propènyle et butadiényle. Le terme halo signifie chloro, bromo, iodo ou fluoro.

L'invention a plus particulièrement pour objet une composition ou un produit tel que défini ci-dessus, caractérisé en ce que
- l'inhibiteur de NO synthase est choisi parmi la L-nitro-arginine (LNA), l'aminoguanidine, le 7-nitro-indazole.
- le piègeur de formes réactives de l'oxygène est choisi parmi le l'acide 3-5-di-tert-butyl-4-hydroxybenzoique.

Les composés inhibiteurs de NO synthase et piègeurs de formes réactives de l'oxygène sont commerciaux ou peuvent être préparés par les méthodes connus de l'homme de l'art (ou par analogie à ces dernières) (P. Hamley et al, Bioorganic and medicinal chemistry letters, vol.5 (15), 1573-1576 (1995) ; W. M. Moore et al, J. Med. Chem., 39, 669-672 (1996) ; E. P. Garvey et al., The Journal of Biological Chemistry, vol.269(43), 26669-26676 (1994)).

Les exemples suivants sont présentés pour illustrer les procédures ci-dessus et ne doivent en aucun cas être considérés comme une limite à la portée de l'invention.

### PARTIE EXPÉRIMENTALE :

Soit A l'inhibiteur de NO synthase et B le piègeur de formes réactives de l'oxygène.

### Exemple 1

Composé AB, combinaison des principes actifs A et B sous forme de sel, dans lequel A et B sont en quantité équimolaire avec comme composé A : l'aminoguanidine, inhibiteur des NO synthases inductibles, et comme composé B : l'acide 3-5-di-tert-butyl-4-hydroxybenzoique, antioxydant piégeur de radicaux libres oxygénés.

### Exemple 2

Composé AB, combinaison des principes actifs A et B sous forme séparée, avec comme composé A : le 7-nitroindazole, inhibiteur des NO synthases constitutives de type neuronal, et comme composé B : l'acide 3-5-di-tert-butyl-4-hydroxybenzoique, antioxydant piégeur de radicaux libres oxygénés.

### Exemple 3

Composé AB, combinaison des principes actifs A et B sous forme séparée et administrée par des voies différentes, avec comme composé A : la N^{G}nitro-arginine, inhibiteur puissant des NO synthases constitutives et inductibles, et comme composé B : l'acide 3-5-di-tert-butyl-4-hydroxybenzoique, antioxydant piégeur de radicaux libres oxygénés.

### Etude pharmacologique des produits de l'invention

Les composés de l'invention ont été soumis à quelques tests biologiques *in vitro* et *in vivo*, afin de prouver leur activité à bloquer la NO synthase (constitutive et inductible) et à pièger les radicaux libres. Leur activité a été évaluée sur un modèle d'ischémie cérébrale focale chez le rat. Dans ce modèle considéré comme un modèle d'infarctus cérébral sévère proche de la situation clinique chez l'homme, une libération excessive de monoxyde d'azote et de formes réactives de l'oxygène a été démontrée ainsi que l'effet partiellement protecteur d'inhibiteur de NO synthase ou de piégeur des formes réactives de l'oxygène. Les effets de l'association ont été comparés à ceux produits par un traitement avec l'inhibiteur de monoxyde d'azote ou le piégeur des formes réactives de l'oxygène seul. L'association d'un inhibiteur de NO synthase et d'un piégeur des formes réactives de l'oxygène montre un effet protecteur hautement significatif sur l'ischémie cérébrale focale alors que l'effet de l'inhibiteur de NO synthase ou du piégeur des formes réactives de l'oxygène pris séparément et aux doses utilisées n'est pas significatif. Ceci prouve la synergie entre l'inhibiteur de NO synthase et le piégeur des formes réactives de l'oxygène.

### 1) Effet in vitro sur la NO synthase constitutive de cervelet de rat

Le test consiste à mesurer la transformation par la NO synthase de la L-arginine en L-citrulline. Des cervelets de rats Sprague-Dawley (300 g - Charles River) sont prélevés rapidement, disséqués à 4° C et homogénéisés dans un volume de tampon d'extraction (HEPES 50 mM, EDTA 1 mM, pH 7,4, pepstatin A 10 mg/ml, leupeptine 10 mg/ml), centrifugés à 21 000 g pendant 15 min à 4° C. Le dosage se fait dans des tubes à essai en verre dans lesquels sont distribués 100 µl de tampon d'incubation contenant 100 mM d'HEPES, pH 7,4, 2 mM d'EDTA, 2,5 mM de CaCl₂, 2 mM de dithiotréitol, 2 mM de NADPH réduit et 10 µg/ml de calnioduline. On ajoute 25 µl d'une solution contenant 100 nM d'arginine tritiée (activité spécifique : 56,4 Ci/mmole, Amersham) et 40 µM d'arginine non radioactive. La réaction est initiée en ajoutant 50 µl d'homogénat, le volume final étant de 200 µl (les 25 µl manquants sont soit de l'eau, soit le produit testé). Après 15 min, la réaction est stoppée avec 2 ml de tampon d'arrêt (20 mM d'HEPES, pH 5,5, 2 mM d'EDTA). Après passage des échantillons sur une colonne de 1 ml de résine DOWEX, la radioactivité est quantifiée par un spectromètre à scintillation liquide. Les résultats sont exprimés en valeur de CI₅₀ et sont résumés dans le tableau du paragraphe 2 (première colonne de résultats intitulée "NO synthase constitutive, formation de citrulline").

### 2) Effet in vitro sur la NO synthase inductible de macrophages murins J774A1

Les macrophages murins J774A1 produisent une grande quantité de monoxyde d'azote après activation par les lipopolysaccharides (LPS) et l'interféron-γ (IFN-γ). Les cellules sont cultivées dans du milieu DMEM (Dulbecco's Modified Eagle' Médium) enrichi avec 10 % de sérum de veau foetal à 37° C sous une atmosphère de 5 % de CO₂ après activation par le LPS et l'IFN-γ. Elles sont ensemencées à raison de 5000 cellules/cm² dans des flacons de 150 cm². Les incubations se font en présence de LPS (1 µg/ml) et d'IFN-γ murin (50 U/ml) dans du DMEM enrichi avec 10 % de sérum de veau foetal. La NO synthase est isolée avec un tampon d'extraction (HEPES 50 mM, pH 7,4, dithiothréitol 0,5 mM, pepstatin A 1 mg/ml, leupeptine 1 mg/ml, inhibiteur de trypsine de soja 1 mg/ml, antipaïne 1 mg/ml et PMSF 10 mg/ml). Après sonication dans le tampon d'extraction à 4° C, les homogénats sont ultra-centrifugés (100 000 g à acide 3-5-di-tert-butyl-4-hydroxybenzoique 4° C pendant 1 h). Le protocole est ensuite identique à celui de la NO synthase de cervelet exceptée la composition du tampon d'incubation (100 mM d'HEPES, pH 7,4, 1 mM de dithiotréitol, 2,5 mM de CaCl₂, 10 µM de tétrahydrobioptérine, FAD 10 µM, BSA 1 mg/ml, 2 mM de NADPH réduit). Les résultats sont exprimés en valeur de CI₅₀ et sont résumés dans le tableau du paragraphe 2 (deuxième colonne de résultats intitulée "NO synthase inductible, formation de citrulline").

### 3) Effet in vitro sur la production de nitrites par les macrophages murins J774A1

Ce test est utilisé pour mesurer l'activité inhibitrice des produits sur la NO synthase inductible de cellules en culture. Les cellules sont cultivées dans du milieu DMEM (Dulbecco's Modified Eagle's Medium) enrichi avec 10 % de sérum de veau foetal à 37° C sous une atmosphère de 5 % de CO₂. Pour les expériences, elles sont réparties en plaques 96 puits (50 000 cellules par puits) et incubées dans du DMEM sans rouge de phénol à 10 % de sérum de veau foetal avec du LPS (1 µg/ml) et de l'IFN-γ murin (50 U/ml) en présence ou en absence des produits à tester. Après 48 heures, la concentration de nitrites dans les milieux de culture, produits de dégradation du monoxyde d'azote, est mesurée par une méthode colorimétrique selon Green et al, (Analytical Biochemistry, 126, 131-138 (1982)). Les résultats sont exprimés en valeur de CI₅₀ et sont résumés dans le tableau du paragraphe 2 (troisième colonne de résultats intitulée "NO synthase inductible, formation de nitrites").

| CI₅₀ (µM) | | | |
|---|---|---|---|
| | NO synthase constitutive (formation de citrulline) | NO synthase inductible (formation de citrulline) | NO synthase inductible (formation de nitrites) |
| Exemple 1 | | | |
| A | > 300 | 29 | 22 |
| AB | > 300 | 57 | 26 |

| Exemple 2 | | | |
|---|---|---|---|
| A | 0,9 | 25 | 51 |
| AB | 0,9 | 25 | 51 |

### 4) Effet in vitro sur la formation des anions superoxydes

L'activation des cellules macrophagiques J774A1 par du phorbol myristate acétate (PMA) entraîne la production d'anions superoxydes en quelques minutes. Ces anions superoxydes peuvent oxyder un substrat luminescent, le luminol. Cette réaction génère des photons dont la production est mesurée à l'aide d'un chemiluminomètre. Les effets des produits sur la production d'anions superoxydes sont testés en incubant les cellules J774A1 à 37° C en présence de PMA, des produits à tester et du luminol. Les résultats sont exprimés par la valeur de CI₅₀.

### Exemple 1

| Produit | CI₅₀ (µM) |
|---|---|
| A | inactif |
| B | 30 |
| AB | 30 |

### 5) Effet sur l'ischémie cérébrale focale avec reperfusion

Les expériences sont réalisées sur des rats mâles Sprague-Dawley (Charles River) pesant entre 330 et 360 g. Après anesthésie à l'isoflurane, les rats sont soumis à une occlusion transitoire de l'artère cérébrale moyenne décrite par Memezawa et al. (Exp. Brain Res. 89, 67-78, 1992). Un fil est introduit dans l'artère cérébrale antérieure provoquant une occlusion de l'artère cérébrale moyenne. Deux heures plus tard, le fil est retiré de manière à permettre la recirculation de sang au niveau du polygone de Willis. La température des animaux est contrôlée et régulée pendant les 6 heures suivant l'occlusion. Quarante-huit heures après l'occlusion, les rats sont anesthésiés à l'isoflurane et décapités. Les cerveaux sont rapidement prélevés et immergés pendant 2 minutes dans de l'isopentane à -15° C. Six coupes de 2 mm d'épaisseur sont réalisées et placées dans une solution à 2 % de chlorure de 2-3-5-triphényltétrazolium (TCC) pendant 20 min et fixées ensuite avec du formalin. La zone infarcie apparaît blanche et la zone saine rouge. La surface infarcie est mesurée au niveau des 6 coupes grâce à un analyseur d'image. Le volume de l'infarctus est calculé en intégrant ces différentes surfaces sachant qu'elles sont distantes de 2 mm. L'administration des produits se fait par voie intrapéritonéale. Le premier traitement se fait 4 heures après l'occlusion, c'est-à-dire, 2 heures après le retrait du fil, puis 24 heures après.

Composé de l'exemple 1 : 4 groupes d'animaux sont constitués :
- Groupe 1 :: traité avec du sérum physiologique ;
- Groupe 2 :: traité avec A (30 mg/kg) ;
- Groupe 3 :: traité avec B (20 mg/kg) ;
- Groupe 4 :: traité avec AB (50 mg/kg).

| N° du groupe | volume de l'infarctus | % de protection |
|---|---|---|
| 1 | 270,4 ± 30,05 | - |
| 2 | 233 ± 30,15 NS | 13,8 |
| 3 | 250,9 ± 37,86 NS | 7,1 |
| 4 | 89,6 ± 22,42 ** | 63,2 |
| (NS: résultat non significatif ; ** : résultat très significatif) | | |

Composé de l'exemple 2 : 4 groupes d'animaux sont constitués :
- Groupe 1 :: traité avec du sérum physiologique ;
- Groupe 2 :: traité avec A (10 mg/kg) ;
- Groupe 3 :: traité avec B (20 mg/kg) ;
- Groupe 4 :: traité avec AB (A : 10 mg/kg et B : 20 mg/kg).

| N° du groupe | volume de l'infarctus | % de protection |
|---|---|---|
| 1 | 270,4 ± 30,05 | - |
| 2 | 238,9 ± 27,08 NS | 11,7 |
| 3 | 250,9 ± 37,86 NS | 7,1 |
| 4 | 69,6 ± 37,86 ** | 74,3 |
| (NS: résultat non significatif ; ** : résultat très significatif) | | |

Composé de l'exemple 3 : 4 groupes d'animaux sont constitués :
- Groupe 1 :: traité avec du sérum physiologique ;
- Groupe 2 :: traité avec A (0,03 mg/kg i.v.);
- Groupe 3 :: traité avec B (20 mg/kg i.p.) ;
- Groupe 4 :: traité avec AB (A: 0,03 mg/kg iv et B : 20 mg/kg ip).

| N° du groupe | volume de l'infarctus | % de protection |
|---|---|---|
| 1 | 237,68 ± 31,51 | - |
| 2 | 238,14 ± 35,94 NS | 0 |
| 3 | 222,9 ± 7,34 NS | 6,2 |
| 4 | 136,5 ± 33,02 ** | 42,6 |
| (NS: résultat non significatif ; ** : résultat très significatif) | | |

Les résultats montrent que l'acide 3-5-di-tert-butyl-4-hydroxybenzoique utilisé comme piégeur des formes réactives de l'oxygène à la dose de 20 mg/kg est inactif pour protéger l'animal d'atteintes ischémiques de même que l'aminoguanidine (inhibiteur de NO synthase) est faiblement active. Par contre, l'association des deux composés protège de manière hautement significative les animaux de l'ischémie.

De la même façon, le 7-nitroindazole utilisé en tant qu'inhibiteur de forme constitutive des NO synthases n'est pas significativement actif à la dose de 10 mg/kg ; par contre, associé à l'acide 3-5-di-tert-butyl-4-hydroxybenzoique, une protection hautement significative est observée. Cet effet protecteur montre une synergie entre les deux principes actifs.

De la même manière, la N^{G} nitro-arginine utilisée en tant qu'inhibiteur des NO synthases et injectée par voie intraveineuse, n'est pas active à la dose de 0,03mg/kg. Par contre, lorsqu'elle est associée à une administration intrapéritonéale d'acide 3-5-di-tert-butyl-4-hydroxybenzoique, une protection hautement significative est observée. Cet effet protecteur montre que la synergie entre les deux principes actifs est aussi observée lorsque les principes actifs sont administrés par des voies différentes.

## Revendications

1. Composition pharmaceutique comprenant, à titre de principe actif, au moins une substance inhibitrice de NO synthase choisie parmi l'aminoguanidine, 7-nitro-indazole et la L-nitro-arginine, et l'acide 3,5-di-tert-butyl-4-hydroxybenzoique en tant que substance piègeur de formes réactives de l'oxygène, et éventuellement un support pharmaceutiquement acceptable.

2. Composition pharmaceutique selon la revendication 1, comprenant, à titre de principe actif, une substance inhibitrice de NO synthase et l'acide 3,5-di-tert-butyl-4-hydroxybenzoique.

3. Composition pharmaceutique selon l'une des revendications précédentes, pour le traitement des ischémies.

## Patentansprüche

1. Pharmazeutische Zusammensetzung, die als Wirkstoff mindestens eine NO-Synthase hemmende Substanz, die aus Aminoguanidin, 7-Nitroindazol und L-Nitroarginin ausgewählt ist, und 3,5-di-tert-Butyl-4-hydroxybenzoesäure als reaktive Formen des Sauerstoffs einfangende Substanz und ggf. einen pharmazeutisch verträglichen Träger enthält.

2. Pharmazeutische Zusammensetzung nach Anspruch 1, die als Wirkstoff eine NO-Synthase hemmende Substanz und 3,5-di-tert-Butyl-4-hydroxybenzoesäure enthält.

3. Pharmazeutische Zusammensetzung nach einem der vorhergehenden Ansprüche für die Behandlung von Ischämien.

## Claims

1. Pharmaceutical composition comprising, as active ingredient, at least one substance inhibiting NO synthase chosen from aminoguanidine, 7-nitroindazole and L-nitroarginine, and the 3,5-di-tert-butyl-4-hydroxybenzoic acid as trap of reactive forms of oxygen, and eventually a pharmaceutical acceptable support.

2. Pharmaceutical composition according to claim 1, comprising, as active ingredient one substance inhibiting NO synthase and the 3,5-di-tert-butyl-4-hydroxybenzoic acid.

3. Pharmaceutical composition according to one of the preceding claims, for the treatment of ischaemia.
